# FASCICULE DE BREVET EUROPEEN

(11) **EP 2 510 904 B1**
(45) Date de publication et mention de la délivrance du brevet: **26.08.2015**
(21) Numéro de dépôt: 12305411.6
(22) Date de dépôt: 06.04.2012
(51) Int. Cl.: A61F 2/44

(54) **Cage intervertebrale pour la fusion**
Bandscheibengerüst für die Fusion
Intervertebral cage for fusion.

(30) Priorité: 11.04.2011 FR 1153121
(43) Date de publication de la demande: 17.10.2012
(73) Titulaire: Les Laboratoires Osteal Medical, 95957 Roissy Aeroport CDG (FR)
(72) Inventeur: Morvan, Stéphane, 13008 MARSEILLE (FR)
(74) Mandataire: Kohler, Pierre

(56) Documents cités:
- EP-A1- 0 880 950
- WO-A2-02/076335
- DE-A1-102005 004 563
- US-A- 6 159 244
- US-A1- 2005 131 536
- US-A1- 2006 247 770
- US-A1- 2006 253 201
- US-B1- 6 217 579

## Description

La présente invention concerne une cage intervertébrale pour la fusion de vertèbres adjacentes en chirurgie de la colonne vertébrale. La fusion des vertèbres est recommandée dans les pathologies dégénératives des disques intervertébraux et se fait en formant un pont osseux entre les deux vertèbres situées de part et d'autre du disque affecté. La cage doit donc être insérée dans l'espace interdiscal qui a souvent dégénéré en un espace très étroit, voire très pincé. La cage contient un substitut osseux facilitant la création du pont osseux entre les vertèbres.

De manière générale, la cage comprend un plateau supérieur destiné à venir en contact avec une vertèbre supérieure et un plateau inférieur destiné à venir en contact avec une vertèbre inférieure. Les plateaux comprennent chacun une ouverture traversante qui crée un espace interne qui s'étend d'une vertèbre à l'autre. Il est également connu dans l'art antérieur de réaliser des plateaux de manière à ce qu'ils puissent se déplacer légèrement l'un par rapport à l'autre entre une position d'insertion rapprochée et une position d'ancrage éloignée. La cage intervertébrale est mise en place entre deux vertèbres en position d'insertion rapprochée, et une fois en place entre les vertèbres, les plateaux sont éloignés l'un de l'autre pour arriver dans la position d'ancrage. Pour ce faire, il est connu d'utiliser une came mobile entre les deux plateaux pour les déplacer de la position d'insertion vers la position d'ancrage. Généralement, la came est déplacée de la face avant vers la face arrière de la cage qui est tourné vers le sac dural. La came est déplacée à travers l'espace interne de la cage de la face avant jusqu'à la face arrière à l'aide d'un ustensile ou ancillaire approprié. Une fois que la came a écarté les plateaux et que la position d'ancrage est atteinte, du substitut osseux est inséré à l'intérieur de l'espace interne de la cage dans le but qu'il vienne en contact avec les deux vertèbres à travers les ouvertures traversantes des plateaux. Une telle cage intervertébrale est notamment décrite dans le document FR-2 914 180. L'opération de remplissage de l'espace interne avec du substitut osseux est une opération délicate et laborieuse pour plusieurs raisons. Tout d'abord, la cage intervertébrale présente de petites dimensions, de l'ordre du centimètre. Ensuite, il n'est pas toujours facile d'accéder à la cage une fois en position d'ancrage entre deux vertèbres. De plus, il n'est pas possible de garantir que l'espace interne est correctement rempli de substitut osseux de manière à venir en contact intime avec les deux vertèbres adjacentes.

La présente invention a pour but de remédier aux inconvénients précités de l'art antérieur en définissant une cage intervertébrale plus facile à manipuler entre les deux vertèbres. Un autre but de la présente invention est d'améliorer le contact entre le substitut osseux et les vertèbres afin de faciliter la création du pont osseux. Un autre but est la reproductibilité du contact entre le substitut osseux et les vertèbres afin de garantir la qualité de la fusion des vertèbres.

Pour atteindre ces buts, la présente invention prévoit une cage intervertébrale pour la fusion de vertèbres adjacentes comprenant un plateau supérieur destiné à venir en contact avec une vertèbre supérieure et un plateau inférieur destiné à venir en contact avec une vertèbre inférieure, les plateaux comprenant chacun une ouverture traversante qui crée un espace interne qui s'étend d'une vertèbre à l'autre, une came mobile entre les deux plateaux entre une position initiale d'insertion et une position finale d'ancrage, un substitut osseux déformable occupant l'espace interne de manière à venir en contact avec les vertèbres pour créer un pont osseux, le substitut osseux étant présent dans l'espace interne en position d'insertion, **caractérisée en ce que** les plateaux sont en contact l'un de l'autre au niveau d'une face avant de la cage et séparés l'un de l'autre sur une face arrière opposée à la face avant, la came étant mobile à partir de la face arrière vers la face avant.

En d'autres termes, le substitut osseux est déjà en place dans l'espace interne de la cage lorsque celle-ci est insérée entre les deux vertèbres. On évite ainsi la manipulation difficile et laborieuse de remplissage de l'espace interne avec du substitut osseux, alors que la cage est déjà en place entre les deux vertèbres. Avantageusement, la came est mobile entre les vertèbres de manière à pousser le substitut osseux hors des ouvertures traversantes des plateaux en contact intime avec les vertèbres pour favoriser la création du pont osseux. En d'autres termes, la came est mobile entre les vertèbres de manière à réduire le volume utile de l'espace interne, forçant ainsi le substitut osseux hors des ouvertures traversantes des plateaux en contact intime avec les vertèbres pour favoriser la création du pont osseux. La came remplit ainsi une fonction de poussée sur le substitut osseux déjà présent dans la cage pour le déformer de manière à fluer ou être « extrudé » à travers les ouvertures latérales des plateaux contre les vertèbres. Cette poussée sur le substitut osseux à l'aide de la came peut s'effectuer avec une réduction ou non du volume utile de l'espace interne. En effet, on peut imaginer que la came pousse le substitut osseux à travers les ouvertures traversantes sans pour autant réduire le volume utile de l'espace interne.

La face arrière peut être tournée vers le sac dural, ou inversement. La cage peut même être introduite latéralement entre deux vertèbres indépendamment de son orientation par rapport au sac dural. Selon une autre définition, la cage intervertébrale comprend une face arrière destinée à être orientée vers le sac dural, dans laquelle la came est mobile à partir de la face arrière en éloignement du sac dural. En situation, le chirurgien accède à la cage par la face avant, étant donné que la face arrière est difficilement accessible du fait de la présence du sac dural, un tissu notoirement très délicat. On peut toutefois envisager dans le cadre de l'invention de déplacer la came à partir de la face avant vers la face arrière.

Selon un autre aspect de l'invention, les plateaux sont déplaçables en éloignement l'un par rapport à l'autre entre la position d'insertion et la position d'ancrage, la came mobile écartant les plateaux de la position d'insertion vers la position d'ancrage. Selon une forme de réalisation pratique, la cage intervertébrale comprend une face avant et une face arrière destinée à être orientée vers le sac dural, les plateaux étant reliés ensemble de manière élastique au niveau de la face avant à la manière d'une épingle, la came étant mobile de la face arrière vers la face avant avec l'espace interne rempli de substitut osseux. Ainsi, en plus de sa fonction de poussée sur le substitut osseux, la came remplit une seconde fonction d'écartement ou d'éloignement des plateaux pour garantir un ancrage solide de la cage entre les vertèbres. Les fonctions de poussée et d'écartement sont réalisées simultanément en déplaçant la came de la position d'insertion à la position d'ancrage. Dans la position d'insertion, les plateaux sont rapprochés, et il est ainsi plus facile d'insérer la cage entre les deux vertèbres, surtout lorsque l'espace intervertébral est rétréci. Une fois insérée entre les vertèbres, il suffit au chirurgien de déplacer la came dans la position d'ancrage, ce qui a pour effet combiné d'écarter les plateaux pour garantir l'ancrage et de déformer le substitut osseux pour garantir son contact intime avec les vertèbres. Il faut bien noter que le déplacement de la came est réalisé alors que le substitut osseux est déjà en place dans la cage.

Selon un autre aspect de l'invention, la came mobile s'étend sur sensiblement toute la largeur de la face arrière, et comprend avantageusement au moins un cran pour stabiliser la came en position d'insertion et/ou d'ancrage.

Selon un autre aspect pratique, la came mobile comprend un patin de poussée venant en appui sur le substitut osseux pour le déformer. Ainsi, le patin permet de déplacer plus de substitut osseux sur une course réduite.

Selon une autre caractéristique avantageuse de l'invention, la cage intervertébrale comprend en outre des plots d'ancrage déplaçables entre une position initiale rétractée dans laquelle les plots sont sensiblement intégrés au plateau et une position finale étendue dans laquelle les plots font saillie par rapport au plateau pour immobiliser la cage entre les vertèbres, les plots étant déplacés par la came mobile de la position initiale vers la position finale.

Selon un autre aspect de l'invention qui peut être mis en oeuvre indépendamment du fait que la came déforme le substitut osseux ou écarte les plateaux ou déploie des plots d'ancrage, la came mobile peut comprendre au moins une patte de traction sur laquelle on peut exercer une traction pour déplacer la came de la position initiale vers la position finale, la patte étant avantageusement formée de manière monobloc avec le restant de la came. Avantageusement, ladite au moins une patte comprend des crans de blocage. En variante, ladite au moins une patte comprend une amorce de rupture au niveau de laquelle est peut être cassée. De préférence, la came comprend au moins une butée de fin de déplacement lorsqu'elle a atteint la position finale. On peut se servir d'un ustensile ou ancillaire approprié pour saisir et tirer sur les pattes. Selon leur nature, on peut soit les couper, soit les casser une fois la came en position finale en butée de fin de déplacement. La came comprend de préférence deux pattes latérales, mais on peut aussi envisager une seule patte centrale.

L'esprit de l'invention réside dans le fait d'utiliser une came mobile entre les deux plateaux d'une cage intervertébrale pour agir sur le substitut osseux afin d'améliorer son contact avec les vertèbres. Le fait que la cage est ouverte sur la face arrière, puisque les plateaux ne sont pas en contact l'un de l'autre sur cette face, facilite à la fois l'insertion du substitut osseux et la mise en place de la came. Cela permet aussi de saisir la came sur ses bords latéraux de chaque côté de la cage pour la déplacer entre les plateaux. Additionnellement, le déplacement de la came peut servir à écarter les plateaux pour augmenter l'ancrage de la cage entre les vertèbres. En outre, le déplacement de la came peut servir à déployer des moyens d'ancrage supplémentaires qui viennent en contact avec les vertèbres. La came mobile peut ainsi remplir une, deux ou trois fonction(s) en même temps. Grâce à l'invention, le substitut osseux peut être mis en place de manière industrielle et contrôlée dans l'espace interne de la cage avant son implantation entre deux vertèbres. Il s'agit là d'un avantage considérable, puisque le chirurgien n'a plus besoin de remplir la cage de substitut osseux, et que le déplacement prédéterminé de la came assure une déformation contrôlée et précise du substitut osseux en contact appuyé contre les vertèbres.

L'invention sera maintenant plus amplement décrite en référence aux dessins joints, donnant à titre d'exemples non limitatifs, deux modes de réalisation de l'invention.

Sur les figures :
La figure 1 est une vue en perspective d'une cage intervertébrale selon un premier mode de réalisation de l'invention,
La figure 2a est une vue latérale de la cage de la figure 1 en position initiale d'insertion,
La figure 2b est une similaire à celle de la figure 2a en position finale d'ancrage,
Les figures 3a et 3b sont des vues en perspective d'une cage selon un second mode de réalisation de l'invention, respectivement en position initiale d'insertion et en position finale d'ancrage,
Les figures 4a et 4b sont des vues en perspective découpées correspondant respectivement aux figures 3a et 3b,
Les figures 5a et 5b sont des vues en perspective éclatée sous deux angles de vue différents de la cage du second mode de réalisation,
La figure 6 est vue en perspective d'une cage intervertébrale selon un troisième mode de réalisation de l'invention ayant une came à pattes, et
La figure 7 est vue en perspective d'une variante de came à pattes.

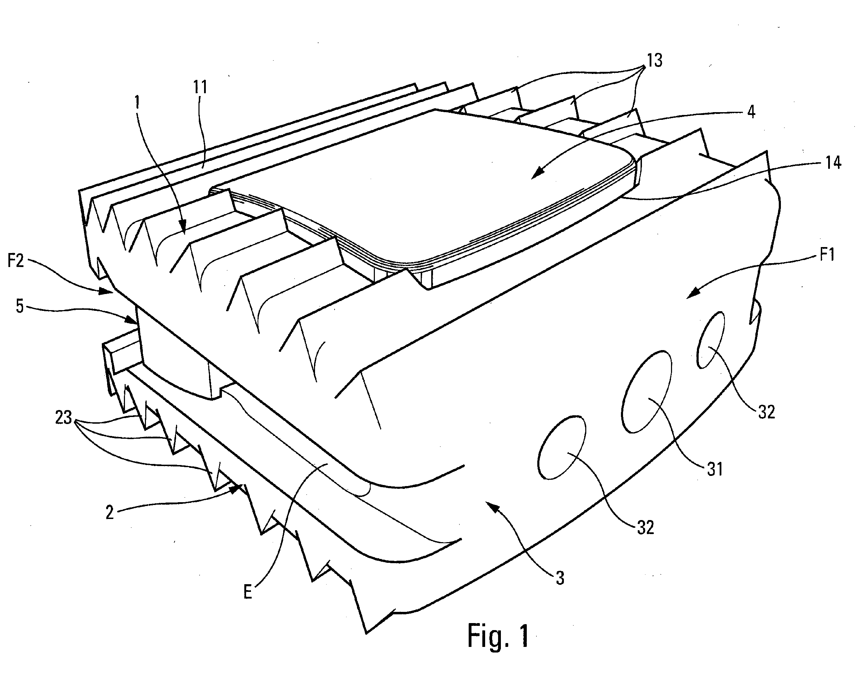

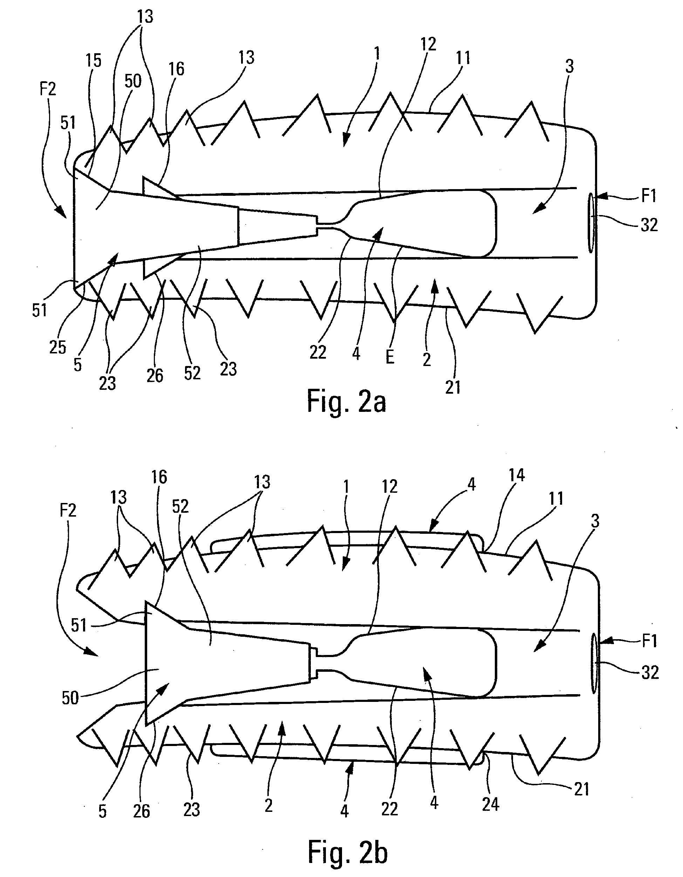

Dans les deux modes de réalisation illustrés sur les figures, les cages intervertébrales présentent une configuration globale sensiblement similaire. En effet, elles comprennent chacune deux plateaux, à savoir un plateau supérieur 1 destiné à venir en contact avec une vertèbre supérieure et un plateau inférieur 2 destiné à venir en contact avec une vertèbre inférieure. Le plateau supérieur 1 comprend une face supérieure 11 qui est avantageusement dotée de profils d'ancrage 13, sous la forme de nervures pointues. A la place de ces nervures pointues, on peut également envisager des profils d'ancrage sous la forme de plots, de dents, etc. Bien entendu, il en est de même pour le plateau inférieur 2 qui comprend une face inférieure 21 pourvue de profils d'ancrage 23 sous la forme de nervures pointues. Le plateau 1 définit également une face inférieure 12 orientée vers le plateau 2 qui forme une face supérieure 22. Les deux plateaux peuvent présenter une configuration sensiblement identique par symétrie miroir. Mais de préférence, les deux plateaux présentent une configuration propre différente, qui est clairement visible sur les figures 2a et 2b. On peut ainsi voir que la face supérieure 11 du plateau supérieur 1 est convexe alors que la face inférieure 21 du plateau inférieur 2 est concave. On peut également remarquer que le plateau supérieur 1 présente une épaisseur supérieure à celle du plateau inférieur 2. Bien entendu, ces caractéristiques ne sont pas critiques pour la présente invention et ne saurait en limiter la portée. Le plateau supérieur 1 est en outre pourvu d'une ouverture traversante 14. Il en est de même pour le plateau inférieur 2 qui est pourvu d'une ouverture traversante 24. Les ouvertures 14, 24 étant traversantes, elles s'étendent des faces supérieures 11, 22 aux faces inférieures 12, 21. Les deux plateaux 1 et 2 sont reliés ensemble par un pont 3 qui s'étend sur un bord des plateaux, comme visible sur les figures 2a et 2b. Le pont 3 peut être traversé de plusieurs trous 31, 32, comme visible sur la figure 1. Le pont 3 peut être relativement souple, de manière à permettre un déplacement relatif des plateaux entre eux. En variante, le pont 3 peut être parfaitement rigide de sorte que les plateaux 1, 2 ne peuvent pas se déplacer l'un par rapport à l'autre. Comme on peut le voir sur les figures 2a et 2b, les deux plateaux 1, 2 sont séparés l'un de l'autre de manière à définir entre eux un espace interne E qui traverse la cage de part en part au niveau des ouvertures 14 et 24. Pour fixer son orientation, on peut définir une face avant F1 de la cage qui est située du côté du pont 3 et une face arrière F2 qui est située sur la face opposée à la face F1. La face arrière F2 est normalement destinée à être orientée vers le sac dural du rachis, c'est-à-dire vers le dos du patient, alors que la face avant F1 est tournée vers la face avant du patient. Avec cette orientation, on peut dire que l'espace interne E est ouvert sur les faces latérales et la face arrière F2 de la cage ainsi qu'au niveau des deux ouvertures 14 et 24, et fermé au niveau de la face avant F1, hormis à travers les trous 31 et 32.

Les deux plateaux 1, 2 et le pont 3 peuvent être réalisés de manière monobloc par injection moulage de matière plastique, comme par exemple du PEEK (polyétheréthercétone), PEKK (polyéthercétonecétone) ou tout autre matériau dont le module d'élasticité approchant celui de l'os cortical.

La cage contient un substitut osseux 4 qui occupe tout ou partie de l'espace interne E. Le substitut osseux est un matériau déformable, malléable et/ou fluable susceptible de subir une déformation plastique non élastique. Le substitut osseux peut par exemple être constitué d'une allogreffe, autogreffe, d'un biomatériau de type phosphate tricalcique, hydroxyapatite, dense ou poreux, céramique biphasée, protéine ostéo-inductrice (type BMP2 ou facteurs de croissances). Le substitut osseux 4 vient en contact de la face inférieure 12 du plateau 1 et de la face supérieure 22 du plateau 2, du pont 3, et s'étend dans les ouvertures 14 et 24.

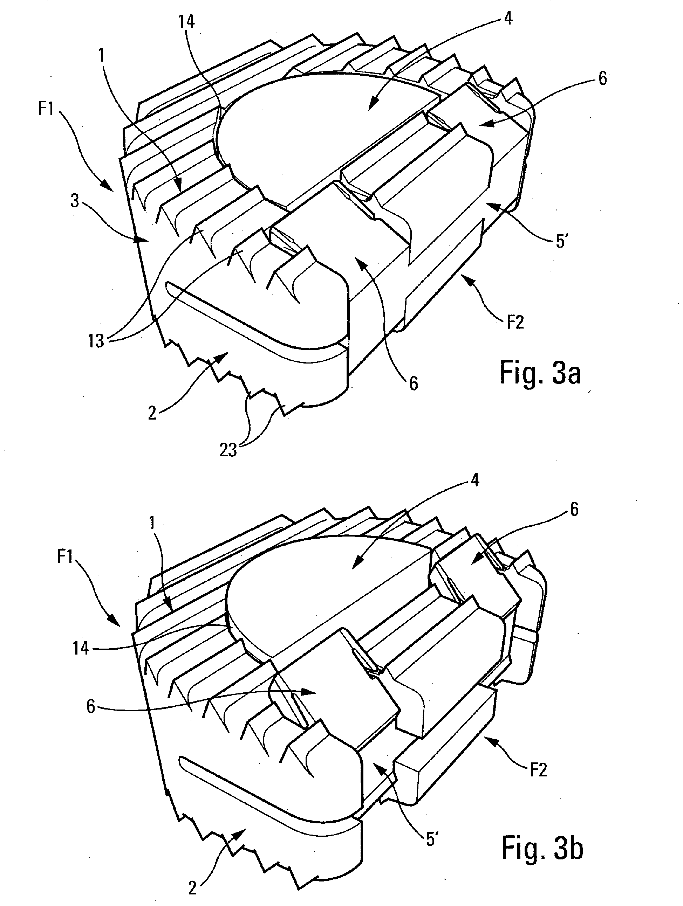

La cage intervertébrale de l'invention, dans les deux modes de réalisation, comprend également une came mobile 5, 5', 5", 5"' qui est engagée entre les deux plateaux 1 et 2 à partir de la face arrière F2. La came peut s'étendre sur toute ou partie de la largeur de la face arrière F2 de la cage. Il est cependant préférable que la came soit légèrement plus courte que la largeur de la face arrière F2 de manière à être encadrée à ses deux extrémités opposées par des parties de plateaux, comme on peut le voir sur les figures 3a et 3b. Il faut noter que la came peut facilement être mise en place entre les plateaux, étant donné qu'ils sont espacés l'un de l'autre sur la face arrière. La configuration générale de la cage en forme d'épingle permet aussi de pouvoir saisir la came par ses bords latéraux pour la déplacer entre les plateaux. La came mobile est déplaçable entre les plateaux 1, 2 entre une position initiale d'insertion représentée sur les figures 2a, 3a et 4a et une position finale d'ancrage représentée sur les figures 2b, 3b et 4b. Avantageusement, le déplacement de la came 5, 5', 5", 5"' est unidirectionnel de la position initiale vers la position finale, le retour étant impossible. En se référant aux figures 2a et 2b, on peut voir que le déplacement de la came s'effectue sur une courte distance, comparativement à la taille de la cage. Cela implique que la came ne traverse pas l'espace interne E jusqu'au niveau du pont 3. Au contraire, la came, dans sa position finale de la figure 2b pénètre légèrement dans l'espace interne E qui est rempli de substitut osseux 4. La came a ainsi pour effet de pousser le substitut osseux 4 de manière à le déformer à travers les ouvertures traversantes 14 et 24 des plateaux 1 et 2. Dans la position finale de la figure 2b, on peut voir que le substitut osseux 4 fait saillie hors des ouvertures 14, 24 par rapport à la face supérieure 11 et la face inférieure 21. On comprend alors aisément que le substitut osseux 4 va pouvoir venir en contact intime contre les deux vertèbres adjacentes. La déformation du substitut osseux 4 par le déplacement de la came 5 peut induire une diminution du volume utile de l'espace interne E, ou non.

Par conséquent, la came 5, 5', 5", 5"' remplit dans tous les modes de réalisation une fonction de poussée sur le substitut osseux 4 afin de le déformer à travers les ouvertures traversantes 14 et 24.

Dans tous les modes de réalisation, la came présente en section transversale une forme générale de coin, facilitant son insertion entre les deux plateaux.

On se référera maintenant aux figures 1 à 2b pour décrire les détails de conception du premier mode de réalisation de l'invention. La came mobile 5 comprend une tête évasée 50 à laquelle se raccorde une partie plus effilée 52. La tête évasée forme deux arêtes vives 51. En position initiale, la tête évasée 51 est située au même niveau que les bords libres des plateaux 1 et 2 et constitue la face arrière F2. La partie effilée 52 est déjà entièrement insérée entre les plateaux 1 et 2. A partir de cette position, on peut déplacer la came 5 entre les deux plateaux jusqu'à venir en position finale représentée sur la figure 2b. Les plateaux 1 et 2 sont chacun formés avec un pan incliné 15, 25. La tête évasée 50 de la came va glisser sur les pans inclinés 15 et 25 des plateaux pour atteindre sa position finale d'ancrage. On peut aussi remarquer que les plateaux 1 et 2 forment deux gorges d'arrêt 16, 26, respectivement formées dans la face inférieure 12 du plateau 1 et la face supérieure 22 du plateau 2. Les deux arêtes vives 51 de la tête évasée 50 viennent se loger dans ces deux gorges d'arrêt 16, 26, bloquant ainsi de manière définitive la came 5 entre les deux plateaux. Les arêtes vives 51 remplissent ainsi une fonction de crans de blocage.

Dans ce premier mode de réalisation, il faut remarquer que les plateaux 1, 2 ont été déplacés en éloignement l'un de l'autre par la came 5 entre la position initiale d'insertion et la position finale d'ancrage. Ceci est clairement visible en comparant les figures 2a et 2b. Cet éloignement des plateaux 1, 2 est rendu possible, soit par l'élasticité du pont 3, soit par l'élasticité intrinsèque des plateaux, soit par une combinaison des deux. Ainsi, en plus de déformer le substitut osseux 4, la came 5 écarte les plateaux 1 et 2 de manière à faire pénétrer profondément les nervures 13 et 23 dans les vertèbres. L'ancrage de la cage entre les vertèbres est ainsi renforcé. Par conséquent, le simple déplacement de la came 5 de la position initiale vers la position finale permet à la fois d'appuyer fortement le substitut osseux 4 contre les vertèbres et d'enfoncer les nervures 13 et 23 dans les vertèbres.

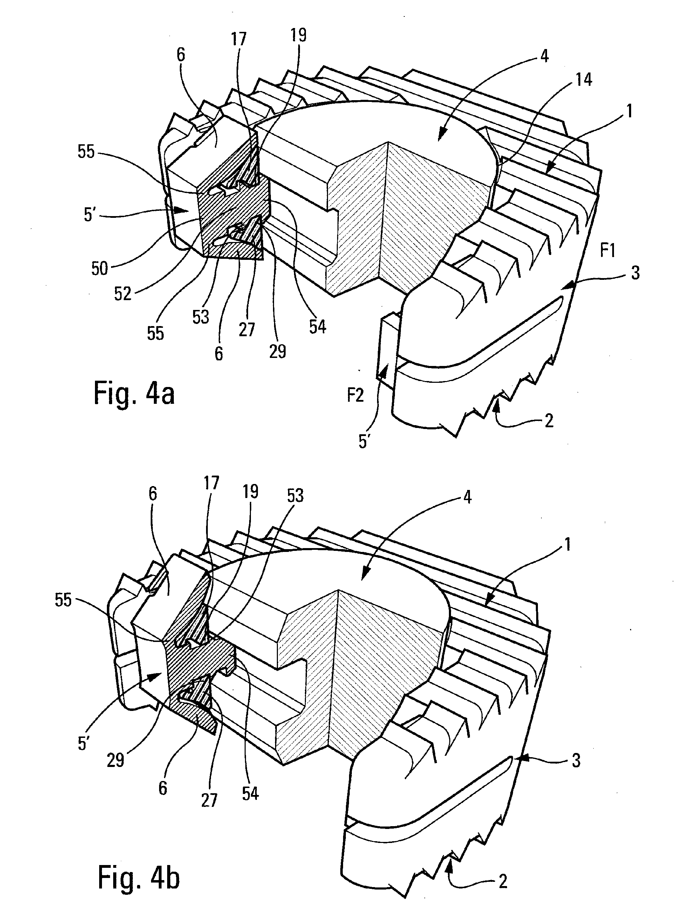

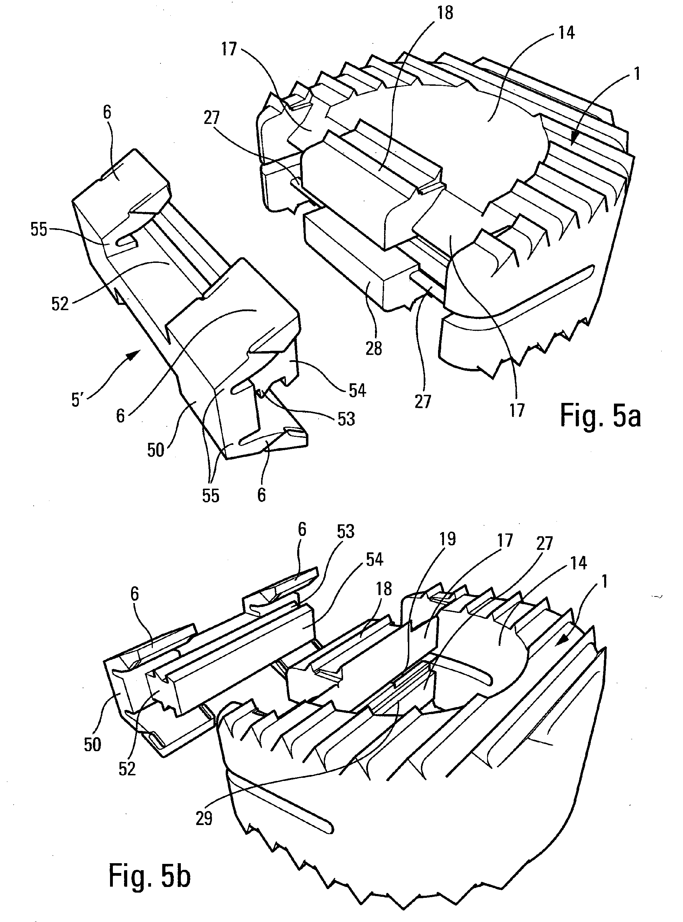

On se référera maintenant aux figures 3a à 5b pour décrire les détails de conception du second mode de réalisation de l'invention. La came mobile 5' remplit également une fonction de poussée permettant de déformer le substitut osseux 4 pour qu'il fasse saillie hors des ouvertures traversantes des plateaux. La came peut également remplir une fonction d'écartement des plateaux, mais cela n'est pas représenté sur les figures 3a à 5b. Il est par conséquent envisageable que la came 5' ne remplisse pas de fonction d'écartement des plateaux. En revanche, la cage est pourvue de plots d'ancrage 6 qui sont déplaçables entre une position initiale rétractée dans laquelle les plots sont sensiblement intégrés aux plateaux et une position finale étendue dans laquelle les plots font saillie par rapport aux plateaux, dans le but d'immobiliser la cage entre les vertèbres. Les plots 6 peuvent se présenter sous la forme de petites languettes qui sont pivotantes entre la position initiale rétractée et la position finale étendue, comme on peut le voir en comparant les figures. Selon l'invention, le déplacement des plots d'ancrage 6 est généré par le déplacement de la came mobile 5' entre la position initiale d'insertion et la position finale d'ancrage. Ceci est clairement visible en comparant les figures 4a et 4b. Ainsi, en plus de sa fonction de poussée sur le substitut osseux 4, la came 5' remplit une fonction d'extension des plots d'ancrage 6, permettant ainsi d'améliorer l'ancrage des plateaux dans les vertèbres. Bien entendu, ces deux fonctions de poussée et d'extension peuvent en outre être cumulées à la troisième fonction d'écartement des plateaux illustrée en référence au premier mode de réalisation de l'invention. Les plots 6 sont ici présents sur les deux plateaux, mais on peut envisager qu'ils ne soient prévus que sur un des plateaux. On peut aussi ne prévoir qu'un seul et unique plot par plateau.

Plus en détail, la came mobile 5' comprend également une tête évasée 50, ainsi qu'une partie plus effilée 52. En outre, la came 5' comprend un patin de poussée 54 qui permet d'augmenter la quantité de substitut osseux poussée lors du déplacement de la came. La came 5' comprend aussi des crans 53 destinés à venir en prise avec des arêtes 19, 29 définies par des barrettes 17, 27 formées respectivement par les deux plateaux 1, 2, dans le but de bloquer la came 5' en position finale et les plots d'ancrage 6 en position étendue. Plus précisément encore, chaque barrette 17, 27 comprend un renfort 18, 28 entre lesquels s'étend la came : les plots 6 sont disposés de part et d'autre de chaque renfort. Les quatre plots d'ancrage 6 sont ici formés de manière monobloc avec la came 5', en étant reliés à celle-ci par un pont de matière 55 faisant charnière. Les plots glissent sur les barrettes 17, 27 qui font office de came de plots avec leur configuration en forme de coin. La came 5' coulissent entre les barrettes 17, 27 pour déformer le substitut osseux 4 avec son patin de poussée 54. Ainsi, le seul déplacement de la came 5' permet à la fois de comprimer le substitut osseux et à déployer les plots d'ancrage 6. Bien que les plots 6 sont ici reliés à la came, on peut imaginer des variantes de réalisation dans lesquelles les plots sont solidaires des plateaux ou même indépendants.

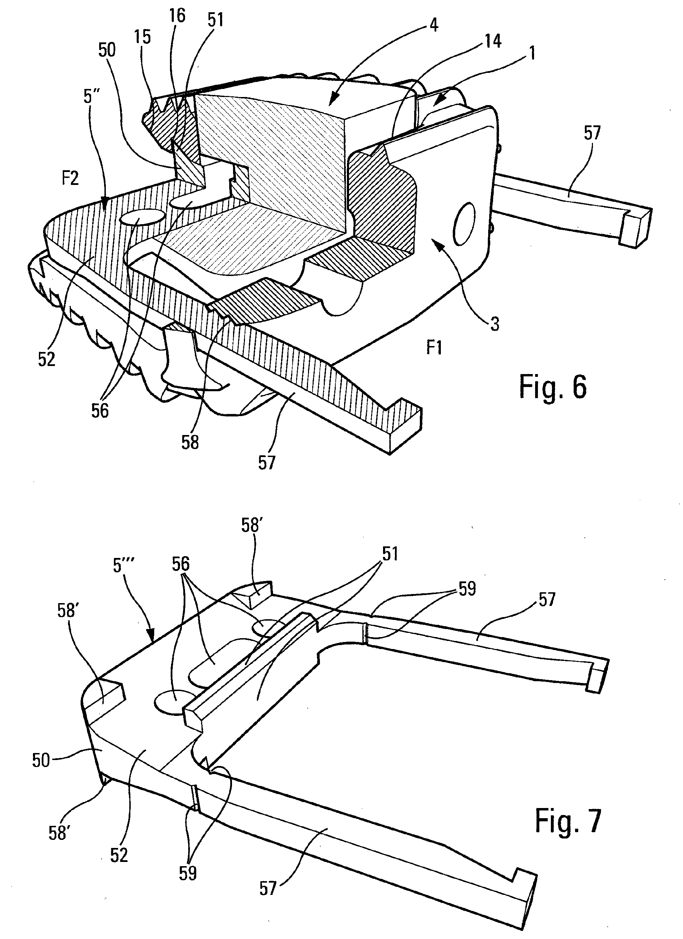

On se référera maintenant à la figure 6 pour décrire les détails de conception du troisième mode de réalisation de l'invention. Tout comme dans les deux modes de réalisation précédents, la came mobile 5" a pour première fonction de comprimer le substitut osseux 4 afin qu'il fasse saillie à travers les ouvertures des plateaux. La came mobile 5" peut également avoir une fonction d'écartement des plateaux comme dans le premier mode de réalisation. Elle peut aussi servir à déployer des plots d'ancrage comme dans le second mode de réalisation. En outre, la came mobile 5" comprend deux pattes de traction latérales 57 qui s'étendent de manière sensiblement parallèle à partir des bords latéraux de la came 5", comme on peut le voir sur la figure 6. Les deux pattes 57 s'étendent ainsi de part et d'autre du substitut osseux 4, et traversent le pont 3 qui relie les deux plateaux ensemble. A ce niveau, les pattes 57 forment des crans de blocage 58, empêchant la came mobile 5" de se déplacer vers la face arrière. De plus, la came 5" comprend deux arrêtes de blocage 51 en prise dans des logements de blocage 16 formés par les plateaux. Ainsi, une fois que la came mobile 5" a atteint sa position finale, il n'est plus possible qu'elle se déplace en retour de manière à détendre le substitut osseux. On peut remarquer sur la figure 6 que les pattes 57 font saillie sur la face avant F1 par rapport au pont 3. La figure 6 représente la came mobile 5" en position finale. Il est alors possible de sectionner les pattes 57 au niveau du pont 3 afin qu'elles ne fassent plus saillie vers l'avant. Bien entendu, afin d'exercer une traction sur les pattes 57, on peut utiliser tout ustensile ou ancillaire approprié. On peut également remarquer que la came mobile 5" comprend plusieurs évidements traversants 56 dans lesquels le substitut osseux 4 peut pénétrer pour favoriser la formation du pont osseux. A la place de deux pattes de traction latérales 57, on peut prévoir une seule patte de traction centrale traversant par exemple le pont 3 de manière centrale. Dans ce cas, la patte de traction unique traverse le substitut osseux 4.

On se référera finalement à la figure 7 qui montre un quatrième mode de réalisation pour une came mobile selon l'invention. Cette came mobile 5"' comprend également deux pattes de traction 57 que l'on peut saisir à l'aide d'un ustensile ou ancillaire approprié pour déplacer la came mobile de sa position initiale vers sa position finale de compression de substitut osseux 4. La came mobile 5"' comprend deux arêtes de butée 51 susceptibles de coopérer avec les deux plateaux pour bloquer la came en position finale et l'empêcher de revenir vers sa position initiale. En outre, la came 5"' comprend quatre butées de fin de déplacement 58' destinées à venir en contact de butée avec les plateaux pour empêcher la came de se déplacer plus en avant lorsqu'elle a atteint sa position finale de compression du substitut osseux. Ainsi, la came 5"' ne peut se déplacer ni en avant ni en arrière lorsqu'elle a atteint sa position finale. On peut remarquer que les pattes de traction 57 sont chacune pourvues d'une amorce de rupture 59 permettant de séparer les pattes 57 de la came 5"', lorsque l'on exerce une traction suffisante. Il n'y a aucun risque de déplacer la came 5"', étant donné qu'elle est bloquée par ces butées de fin de course 58'. Ainsi, il suffit à l'opérateur de tirer sur les pattes 57 jusqu'à ressentir un point de résistance qui correspond à la position finale. A partir de là, en continuant à tirer sur les pattes 57, elles vont se détacher de la came 5"' au niveau des amorces de rupture 59. Au lieu d'avoir deux pattes de traction 57, on peut également imaginer une came ne comprenant qu'une seule patte de traction centrale qui traverse le substitut osseux 4.

Il est à noter que les pattes de traction 57 des figures 6 et 7 peuvent être mises en oeuvre sur n'importe quelle came mobile, indépendamment du fait qu'elle exerce une fonction de compression du substitut osseux, d'écartement des plateaux ou de déploiement de plots d'ancrage. Par conséquent, une protection peut être recherchée spécifiquement sur une came quelconque comprenant une ou deux patte(s) de traction 57.

Dans tous les modes de réalisation, la came mobile est déplacée de manière à pousser ou comprimer le substitut osseux hors des ouvertures pratiquées dans les plateaux. Le déplacement de la came est effectué à partir de la face arrière F2 vers la face avant F1 à l'intérieur de l'espace interne E. Bien que non représenté, il est également possible de déplacer la came à partir de la face avant F1 vers la face arrière F2, particulièrement dans le cas où la came ne remplit pas de fonction d'écartement des plateaux. Il n'est pas non plus exclu de déplacer la came latéralement.

Grâce à l'invention, non seulement le substitut osseux peut être monté en série dans la cage avant son implantation, mais encore il peut être déformé de manière contrôlée en déplaçant la came, qui peut en outre remplir d'autres fonctions complémentaires, comme celles d'écartement des plateaux ou d'extension de moyens d'ancrage. Le fait que les plateaux ne soient reliés que sur la face avant facilite l'insertion du substitut osseux entre les plateaux, la mise en place de la came entre les plateaux et la préhension ou traction de la came par ses bords latéraux de chaque côté de la cage.

## Revendications

1. Cage intervertébrale pour la fusion de vertèbres adjacentes comprenant :
- un plateau supérieur (1) destiné à venir en contact avec une vertèbre supérieure et un plateau inférieur (2) destiné à venir en contact avec une vertèbre inférieure, les plateaux (1, 2) comprenant chacun une ouverture traversante (14, 24) qui crée un espace interne (E) qui s'étend d'une vertèbre à l'autre,
- une came mobile (5 ; 5' ; 5", 5"') entre les deux plateaux (1, 2) entre une position initiale d'insertion et une position finale d'ancrage,
- un substitut osseux déformable (4) occupant l'espace interne (E) de manière à venir en contact avec les vertèbres pour créer un pont osseux, le substitut osseux (4) étant présent dans l'espace interne (E) en position d'insertion,
**caractérisée en ce que** les plateaux (1, 2) sont en contact l'un de l'autre au niveau d'une face avant (F1) de la cage et séparés l'un de l'autre sur une face arrière (F2) opposée à la face avant (F1), la came (5 ; 5' ; 5", 5"') étant mobile à partir de la face arrière (F2) vers la face avant (F1).

2. Cage intervertébrale selon la revendication 1, dans laquelle la came (5 ; 5' ; 5", 5"') est mobile entre les plateaux (1, 2) de manière à pousser le substitut osseux (4) hors des ouvertures traversantes (14, 24) des plateaux (1, 2) en contact intime avec les vertèbres pour favoriser la création du pont osseux.

3. Cage intervertébrale selon la revendication 1 ou 2, dans laquelle la came (5 ; 5' : 5", 5"') est mobile entre les plateaux (1, 2) de manière à réduire le volume utile de l'espace interne (E), forçant ainsi le substitut osseux (4) hors des ouvertures traversantes (14, 24) des plateaux (1, 2) en contact intime avec les vertèbres pour favoriser la création du pont osseux.

4. Cage intervertébrale selon l'une quelconque des revendications précédentes, dans laquelle les plateaux (1, 2) sont déplaçables en éloignement l'un par rapport à l'autre entre la position d'insertion et la position d'ancrage, la came mobile (5 ; 5" ; 5"') écartant les plateaux (1, 2) de la position d'insertion vers la position d'ancrage.

5. Cage intervertébrale selon l'une quelconque des revendications précédentes, comprenant une face avant (F1) et une face arrière (F2) destinée à être orientée vers le sac dural, les plateaux (1, 2) étant reliés ensemble de manière élastique au niveau de la face avant (F1) à la manière d'une épingle, la came (5 ; 5" ; 5"') étant mobile de la face arrière (F2) vers la face avant (F1) avec l'espace interne (E) rempli de substitut osseux (4).

6. Cage intervertébrale selon l'une quelconque des revendications précédentes, dans laquelle la came mobile (5 ; 5' ; 5", 5"') s'étend sur sensiblement toute la largeur de la face arrière (F2), et comprend avantageusement au moins un cran (51 ; 53 ; 58 ; 58') pour stabiliser la came (5 ; 5' ; 5", 5"') en position d'insertion et/ou d'ancrage.

7. Cage intervertébrale selon l'une quelconque des revendications précédentes, dans laquelle la came mobile (5') comprend un patin de poussée (54) venant en appui sur le substitut osseux (4) pour le déformer.

8. Cage intervertébrale selon l'une quelconque des revendications précédentes, comprenant en outre des plots d'ancrage (6) déplaçables entre une position initiale rétractée dans laquelle les plots (6) sont sensiblement intégrés au plateau (1, 2) et une position finale étendue dans laquelle les plots (6) font saillie par rapport au plateau (1, 2) pour immobiliser la cage entre les vertèbres, les plots (6) étant déplacés par la came mobile (5') de la position initiale vers la position finale.

9. Cage intervertébrale selon l'une quelconque des revendications précédentes, dans laquelle la came mobile (5" ; 5"') comprend au moins une patte de traction (57) sur laquelle on peut exercer une traction pour déplacer la came de la position initiale vers la position finale, la patte (57) étant avantageusement formée de manière monobloc avec le restant de la came.

10. Cage intervertébrale selon la revendication 9, dans laquelle ladite au moins une patte (57) comprend des crans de blocage (58).

11. Cage intervertébrale selon la revendication 9, dans laquelle ladite au moins une patte (57) comprend une amorce de rupture (59) au niveau de laquelle est peut être cassée.

12. Cage intervertébrale selon la revendication 11, dans laquelle la came (5"') comprend au moins une butée de fin de déplacement (58') lorsqu'elle a atteint la position finale.

## Patentansprüche

1. Bandscheibengerüst für die Fusion von nebeneinander liegenden Rückenwirbeln, inklusive:
- einer oberen Platte (1), dafür bestimmt, mit einem oberen Rückenwirbel in Berührung zu kommen und einer unteren Platte (2), dafür bestimmt, mit einem unteren Rückenwirbel in Berührung zu kommen, die Platten (1, 2), die jeweils eine Durchführungsöffnung (14, 24) aufweisen, die einen Innenbereich (E) schaffen, der sich von einem Rückenwirbel zum anderen erstreckt,
- einer mobilen Stufenscheibe (5; 5'; 5"; 5"') zwischen den beiden Platten (1, 2) zwischen einer ursprünglichen Einsetzposition und einer finalen Befestigungsposition,
- eines verformbaren Knochenersatzes (4), der den internen Bereich (E) so ausfüllt, dass er mit den Rückenwirbeln in Kontakt kommt, um eine Knochenbrücke zu bilden, der Knochenersatz (4) ist im internen Bereich (E) in der Einsetzposition vorhanden,
**gekennzeichnet dadurch, dass** sich die Platten (1, 2) auf Höhe der Vorderseite (F1) eines jeden Bandscheibengerüsts berühren und auf der Rückseite (F2) des Bandscheibengerüsts voneinander getrennt sind, auf der Vorderseite (F1) ist die Stufenscheibe (5; 5'; 5"; 5"') ab der Hinterseite (F2) bis zur Vorderseite (F1) beweglich.

2. Bandscheibengerüst nach Anspruch 1, in dem die Stufenscheibe (5; 5'; 5"; 5"') zwischen den Platten (1, 2) so beweglich ist, dass sie den Knochenersatz (4) gegen die Durchführungsöffnungen (14, 24) der Platten (1, 2) drücken, mit engem Kontakt zu den Rückenwirbeln, um die Bildung der Knochenbrücke zu begünstigen.

3. Bandscheibengerüst nach Anspruch 1 oder 2, in dem die Stufenscheibe (5; 5'; 5"; 5"') zwischen den Platten (1, 2) so beweglich ist, dass das Nutzungsvolumen des Innenbereichs (E) reduziert wird, somit wird der Knochenersatz (4) gegen die Durchführungsöffnungen (14, 24) der Platten (1, 2) gedrückt und es wird enger Kontakt zu den Rückenwirbeln geschaffen, um die Bildung der Knochenbrücke zu begünstigen.

4. Bandscheibengerüst nach einem der oben genannten Ansprüche, in dem die Platten (1, 2) verschiebbar sind, durch Trennung in Bezug auf die andere zwischen der Position des Einsetzens und der Position der Befestigung, die mobile Stufenscheibe (5; 5"; 5"') verschiebt dabei die Platten (1, 2) von der Position des Einsetzens bis zur Position der Befestigung.

5. Bandscheibengerüst nach einem der oben genannten Ansprüche, das eine Vorderseite (F1) und eine Rückseite (F2) umfasst, die dafür bestimmt ist, in Richtung des Duralsacks ausgerichtet zu werden, die Platten (1, 2) sind somit miteinander elastisch auf Höhe der Vorderseite (F1) verbunden, wie eine Art Nadel, die Stufenscheibe (5; 5"; 5"') ist somit von der Vorderseite (F2) in Richtung Vorderseite (F1) beweglich, wobei der Innenbereich (E) mit dem Knochenersatz (4) ausgefüllt ist.

6. Bandscheibengerüst nach einem der oben genannten Ansprüche, in dem die bewegliche Stufenscheibe (5; 5'; 5"; 5"') sich deutlich über die gesamte Breite der Rückseite (F2) erstreckt und vorteilhaft mindestens einen Einschnitt (51; 53; 58; 58') umfasst, um die Stufenscheibe (5; 5'; 5"; 5"') in der Position des Einsetzens und/oder der Befestigung zu stabilisieren.

7. Bandscheibengerüst nach einem der oben genannten Ansprüche, in dem die bewegliche Stufenscheibe (5') einen Stoßpuffer (54) umfasst, der auf dem Knochenersatz (4) aufliegt, um diesen zu deformieren.

8. Bandscheibengerüst nach einem der oben angegebenen Ansprüche, das außerdem verschiebbare Befestigungskontakte (6) umfasst, die zwischen einer ersten zurückgezogenen Position, in der die Kontakte (6) deutlich in die Platte (1, 2) integriert sind, und einer finalen erweiterten Positionen, in der die Kontakte (6) einen Vorbau in Bezug auf die Platte (1, 2) bilden, verschoben werden können, um das Bandscheibengerüst zwischen den Rückenwirbeln unbeweglich zu halten, die Kontakte (6) werden dabei durch die bewegliche Stufenscheibe (5') aus der ursprünglichen Position in die finale Position verschoben.

9. Bandscheibengerüst nach einem der oben genannten Ansprüche, in der die bewegliche Stufenscheibe (5", 5') mindestens eine Zuglasche (57) umfasst, über die man Zug ausüben kann, um die Stufenscheibe von der ursprünglichen Position in die finale Position zu verschieben, die Lasche (57) ist verteilhaft als Monoblock mit dem Rest der Stufenscheibe geformt.

10. Bandscheibengerüst nach Anspruch 9, in dem besagte mindestens vorhandene Lasche (57) die Blockierungseinschnitte (58) umfasst.

11. Bandscheibengerüst nach Forderung 9, indem besagte mindestens vorhandene Lasche (57) einen Vorriss (59) umfasst, auf dessen Höhe diese gebrochen werden kann.

12. Bandscheibengerüst nach Anspruch 11, in dem die Stufenscheibe (5"') mindestens eine Verschiebungsbegrenzung (58'), bis sie die finale Position erreicht, umfasst.

## Claims

1. An intervertebral cage for the fusion of adjacent vertebrae comprising:
- an upper plate (1) intended to come into contact with an upper vertebra and a lower plate (2) intended to come into contact with a lower vertebra, the plates (1, 2) each comprising a through-aperture (14, 24) which creates an inner space (E) which extends from one vertebra to the other,
- a movable cam (5; 5'; 5" , 5"') between both plates (1, 2) between an initial insertion position and a final anchoring position,
- a deformable bone substitute (4) occupying the inner space (E) so as to come into contact with the vertebrae for creating a bone bridge, the bone substitute (4) being present in the inner space (E) in the insertion position,
**characterized in that** the plates (1, 2) are in contact with each other at a front face (F1) of the cage and separated from each other on a rear face (F2) opposite to the front face (F1), the cam (5; 5' ; 5", 5"') being movable from the rear face (F2) to the front face (F1).

2. The intervertebral cage according to claim 1, wherein the cam (5; 5'; 5", 5"') is movable between the plates (1, 2) so as to push the bone substitute (4) out of the through-apertures (14, 24) of the plates (1, 2) in intimate contact with the vertebrae in order to promote creation of the bone bridge.

3. The intervertebral cage according to claim 1 or 2, wherein the cam (5; 5'; 5", 5"') is movable between the plates (1, 2) so as to reduce the useful volume of the inner space (E), thereby forcing the bone substitute (4) out of the through-apertures (14, 24) of the plates (1, 2) in intimate contact with the vertebrae in order to promote creation of the bone bridge.

4. The intervertebral cage according to any one of the preceding claims, wherein the plates (1, 2) are displaceable away from each other between the insertion position and the anchoring position, the movable cam (5; 5", 5"') moving away the plates (1, 2) from the insertion position to the anchoring position.

5. The intervertebral cage according to any one of the preceding claims, comprising a front face (F1) and a rear face (F2) intended to be oriented towards the dural sac, the plates (1, 2) being connected together elastically at the front face (F1) like a pin, the cam (5; 5", 5"') being movable from the rear face (F2) to the front face (F1) with the inner space (E) filled with bone substitute (4).

6. The intervertebral cage according to any one of the preceding claims, wherein the movable cam (5; 5'; 5", 5"') extends over substantially the whole width of the rear face (F2), and advantageously comprises at least one notch (51; 53; 58; 58') for stabilizing the cam (5; 5'; 5", 5"') in the insertion and/or anchoring position.

7. The intervertebral cage according to any one of the preceding claims, wherein the movable cam (5') comprises a thrust pad (54) which will bear upon the bone substitute (4) in order to deform it.

8. The intervertebral cage according to any one of the preceding claims, further comprising anchoring studs (6) displaceable between an initial retracted position in which the studs (6) are substantially integrated to the plate (1, 2) and an extended final position in which the studs (6) protrude from the plate (1, 2) for immobilizing the cage between the vertebrae, the studs (6) being displaced by the movable cam (5') from the initial position to the final position.

9. The intervertebral cage according to any one of the preceding claims, wherein that the mobile cam (5" ; 5"') comprises at least one pull tab (57) on which traction may be exerted for displacing the cam from the initial position to the final position, the tab (57) being advantageously formed in one piece with the remainder of the cam.

10. The intervertebral cage according to claim 9, wherein said at least one tab (57) comprises blocking notches (58).

11. The intervertebral cage according to claim 9, wherein said at least one tab (57) comprises a weakened line (59) at which it may be broken.

12. The intervertebral cage according to claim 11, wherein the cam (5"') comprises at least one end-of-travel abutment (58') when it has reached the final position.
